(19) **Europäisches Patentamt**
**European Patent Office**
**Office européen des brevets**

(11) **EP 2 238 473 B1**

(12) **FASCICULE DE BREVET EUROPEEN**

(45) Date de publication et mention
de la délivrance du brevet:
**20.03.2013 Bulletin 2013/12**

(21) Numéro de dépôt: **09703572.9**

(22) Date de dépôt: **23.01.2009**

(51) Int Cl.:
**G01T 1/29** (2006.01)

(86) Numéro de dépôt international:
**PCT/EP2009/050748**

(87) Numéro de publication internationale:
**WO 2009/092778 (30.07.2009 Gazette 2009/31)**

(54) **PROCÉDÉ DE LOCALISATION D'UN RADIONUCLÉIDE À POSITONS, APPLICATIONS ET DISPOSITIF DE MISE EN OEUVRE**

VERFAHREN ZUR ORTUNG EINES POSITRONENRADIONUKLIDS, ANWENDUNGEN UND VORRICHTUNG ZUR UMSETZUNG DIESES VERFAHRENS

METHOD FOR LOCATING A POSITON RADIONUCLIDE, APPLICATIONS AND DEVICE FOR IMPLEMENTING SAME

(84) Etats contractants désignés:
**AT BE BG CH CY CZ DE DK EE ES FI FR GB GR HR HU IE IS IT LI LT LU LV MC MK MT NL NO PL PT RO SE SI SK TR**

(30) Priorité: **25.01.2008 FR 0850485**

(43) Date de publication de la demande:
**13.10.2010 Bulletin 2010/41**

(73) Titulaires:
• **Centre National de la Recherche Scientifique (CNRS)**
**75016 Paris (FR)**
• **Ecole Des Mines De Nantes**
**44300 Nantes (FR)**

(72) Inventeurs:
• **CUSSONNEAU, Jean-Pierre**
**F-49270 Le Fuilet (FR)**
• **LE RAY, Patrick**
**F-44800 Saint Herblain (FR)**
• **MORTEAU, Eric**
**F-49600 La Chaussaire (FR)**
• **SERVAGENT, Noël**
**F-44240 La Chapelle sur Erdre (FR)**
• **THERS, Dominique**
**F-44119 Treillieres (FR)**

(74) Mandataire: **Regimbeau**
**Espace Performance Bâtiment K**
**35769 St-Grégoire Cedex (FR)**

(56) Documents cités:
**US-B1- 6 484 051**

• **OBERLACK U G ET AL: "COMPTON SCATTERING SEQUENCE RECONSTRUCTION ALGORITHM FOR THE LIQUID XENON GAMMA-RAY IMAGING TELESCOPE (LXeGRIT)"[Online] 13 décembre 2000 (2000-12-13), pages 1-10, XP002504704 Extrait de l'Internet: URL:http://arxiv.org/PS_cache/astro-ph/pdf /0012/0012296v1.pdf> [extrait le 2008-11-18]**
• **DATABASE COMPENDEX [Online] ENGINEERING INFORMATION, INC., NEW YORK, NY, US; KURFESS J D ET AL: "Coincident Compton nuclear medical imager" XP002504705 Database accession no. E2002357064749 & IEEE NUCLEAR SCIENCE SYMPOSIUM AND MEDICAL IMAGING CONFERENCE 2001 INSTITUTE OF ELECTRICAL AND ELECTRONICS ENGINEERS INC. US, vol. 2, 2001, pages 1166-1170,**
• **GRIGNON ET AL: "Nuclear medical imaging using beta<+>gamma coincidences from <44>Sc radio-nuclide with liquid xenon as detection medium" NUCLEAR INSTRUMENTS & METHODS IN PHYSICS RESEARCH, SECTION - A: ACCELERATORS, SPECTROMETERS, DETECTORS AND ASSOCIATED EQUIPMENT, ELSEVIER, AMSTERDAM, NL, vol. 571, no. 1-2, 26 janvier 2007 (2007-01-26), pages 142-145, XP005737906 ISSN: 0168-9002 cité dans la demande**

**Description**

L'invention concerne un procédé de localisation de la position d'un radionucléide émetteur de positons lors de sa désintégration et dont le noyau fils émet au moins un photon par désexcitation.

Un domaine d'application est la localisation de marqueurs radioactifs par exemple à des fins thérapeutiques, ou autres, telles que par exemple la localisation de marqueurs dans un milieu géologique (sous-sol, roches) ou sur un prélèvement en profondeur dans un milieu géologique (carottes), pour analyser le trajet préférentiel emprunté dans l'écoulement du radioélément, comme par exemple pour déterminer les lieux d'écoulement d'eau de pluie ou d'eaux usées.

Le document [1] indique qu'un tel procédé peut être mis en oeuvre en détectant par une caméra à tomographie par émission de positons (TEP ou PET en anglais) une ligne de réponse du radionucléide.

Selon le document [1], la position du radionucléide est située à l'intersection entre la ligne de réponse et un cône géométrique déterminé à partir d'un télescope Compton, le cône ayant pour sommet la position d'une interaction de type Compton détectée dans un milieu de détection d'interactions Compton du télescope à partir du photon émis par le radionucléide, et pour demi-ouverture l'angle entre la direction du photon incident de l'interaction et l'axe de révolution du cône formé par la direction joignant cette interaction et la position d'une autre interaction détectée par le télescope.

Le document [1] précise qu'il faut utiliser la mesure des trois coordonnées et de l'énergie des deux premières interactions du photon incident, mais omet de décrire comment identifier ces deux premières interactions par le télescope Compton.

Le document US6484051 B1 décrit un procédé selon le préambule de la revendication 1 ainsi qu'un dispositif selon le préambule de la revendication 11.

L'invention vise à obtenir un procédé de localisation du radionucléide apportant des moyens permettant de résoudre ce problème.

Un objet de l'invention est un procédé de localisation de la position d'un radionucléide émetteur de positons et dont le noyau fils émet au moins un photon par désexcitation, dans lequel

on détecte par une caméra à tomographie par émission de positons une ligne de réponse du radionucléide,

la position du radionucléide étant située à l'intersection entre la ligne de réponse et un cône géométrique déterminé, le cône ayant pour sommet la position d'une interaction de type Compton à partir du photon émis par le radionucléide, et pour demi-ouverture l'angle entre la direction du photon incident de l'interaction et l'axe de révolution du cône formé par la direction joignant cette interaction et la position d'une autre interaction,

un télescope Compton est utilisé pour détecter une pluralité, supérieure ou égale à deux, d'interactions de type Compton provoquées en cascade dans un milieu de détection du télescope Compton à partir du photon émis par le radionucléide,

on mesure par le télescope Compton la position de chaque interaction de ladite pluralité,

caractérisé en ce que

pour chacun de la multiplicité des arrangements ordonnés par paire de première et deuxième interactions de type Compton parmi la pluralité d'interactions détectées, on détermine par le télescope Compton l'angle entre la direction du photon incident de la première interaction et l'axe géométrique joignant la position de la première interaction et la position de la deuxième interaction et on reconstruit la surface géométrique du cône ayant pour sommet la position de la première interaction et pour demi-ouverture ledit angle déterminé autour dudit axe géométrique, ledit axe géométrique formant l'axe de révolution du cône et étant orienté dans le sens allant de la deuxième interaction vers la première interaction,

on élimine les cônes dont la surface géométrique ne possède pas d'intersection avec la ligne de réponse, on sélectionne au moins un cône dont la surface géométrique possède une intersection avec la ligne de réponse,

on sélectionne la position du radionucléide à partir de ladite intersection reconstruite de la ligne de réponse avec la surface géométrique du cône sélectionné.

Suivant des modes de réalisation de l'invention :

- On détermine par le télescope Compton ledit angle mn pour chaque arrangement par paire suivant la formule :

$$\cos \theta_{mn} = 1 + m_e c^2 (1/E_{0m} - 1/(E_{0m} - E_{1m}))$$

où $m_e$ est la masse de l'électron, c représente la vitesse de la lumière dans le vide, $E_{0m}$ est l'énergie mesurée du photon incident de la première interaction de la paire, $E_{1m}$ est l'énergie transférée à un électron lors de la première interaction et mesurée.

- On examine si il n'y a qu'un seul cône sélectionné dont la surface possède un point d'intersection avec la ligne de réponse, et, dans l'affirmative, on calcule la position du radionucléide comme étant la position de ce point d'intersection de ce seul cône sélectionné dont la surface possède un point d'intersection avec la ligne de réponse.

- On examine si il y a plusieurs cônes sélectionnés dont la surface possède un point d'intersection avec la ligne de réponse, et, dans l'affirmative, on détermine lequel de ces cônes sélectionnés dont la surface possède un point d'intersection avec la ligne de réponse est le plus probable, on calcule la position du radionucléide comme étant la position du point d'intersection du cône sélectionné, dont la surface possède un point d'intersection avec la ligne de réponse et qui est le plus probable.

D'autres objets de l'invention sont les suivants :

- Application du procédé tel que décrit ci-dessus à l'imagerie de localisation du radionucléide.
- Application du procédé tel que décrit ci-dessus à la localisation d'au moins un marqueur radioactif comprenant ledit radionucléide.
- Cette dernière application peut servir à la localisation d'au moins un marqueur radioactif, comprenant le radionucléide, pour localiser dans un milieu géologique une substance radiomarquée par le marqueur.
- Dans cette dernière application la substance radiomarquée peut être de l'eau.
- Application du procédé tel que décrit ci-dessus à la localisation d'au moins un marqueur radioactif, comprenant le radionucléide, pour marquer une substance chimique dans un corps humain ou animal.
- Application du procédé tel que décrit ci-dessus à la localisation d'au moins un marqueur radioactif, comprenant le radionucléide, pour marquer une substance chimique dans un corps humain ou animal, ayant été introduite dans ledit corps.
- Application du procédé tel que décrit ci-dessus à la détection de fuites d'un container contenant au moins le radionucléide.
- Application du procédé tel que décrit ci-dessus à la détection d'un objet contenant au moins le radionucléide.

Un autre objet de l'invention est un dispositif pour la mise en oeuvre du procédé tel que décrit ci-dessus, comprenant
une caméra à tomographie par émission de positons pour détecter une ligne de réponse du radionucléide,
un télescope Compton comportant :

- des moyens pour détecter une pluralité, supérieure ou égale à deux, d'interactions de type Compton provoquées en cascade dans un milieu de détection du télescope Compton à partir du photon émis par le radionucléide,
- des moyens de mesure de la position de chaque interaction de ladite pluralité dans le milieu de détection,

le dispositif étant caractérisé en ce qu'il comporte, en plus de la caméra et du télescope :

- des moyens agencés pour déterminer, pour chacun de la multiplicité des arrangements ordonnés par paire de première et deuxième interactions de type Compton parmi la pluralité d'interactions détectées, l'angle entre la direction du photon incident de la première interaction et l'axe géométrique joignant la position de la première interaction et la position de la deuxième interaction, pour reconstruire la surface géométrique du cône ayant pour sommet la position de la première interaction et pour demi-ouverture ledit angle déterminé autour dudit axe géométrique, ledit axe géométrique formant l'axe de révolution du cône et étant orienté dans le sens allant de la deuxième interaction vers la première interaction,
- des moyens agencés pour éliminer les cônes dont la surface géométrique ne possède pas d'intersection avec la ligne de réponse,
- des moyens agencés pour sélectionner au moins un cône dont la surface géométrique possède une intersection avec la ligne de réponse,
- des moyens agencés pour sélectionner la position du radionucléide à partir de ladite intersection reconstruite de la ligne de réponse avec la surface géométrique du cône sélectionné.

L'invention sera mieux comprise à la lecture de la description qui va suivre, donnée uniquement à titre d'exemple non limitatif en référence aux dessins annexés, sur lesquels :

- la figure 1 est un schéma montrant les interactions provoquées par un radionucléide se désintégrant, et un dispositif pour la mise en oeuvre du procédé suivant l'invention, et

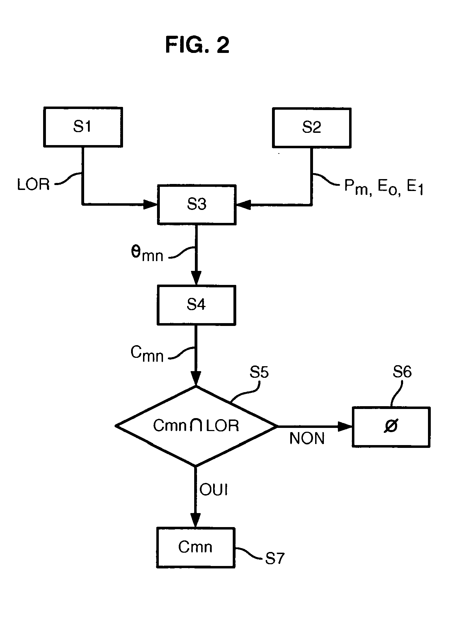

- la figure 2 est un organigramme d'étapes mises en oeuvre dans le procédé suivant l'invention pour reconstituer la séquence Compton provoquée par le radionucléide.

Dans ce qui suit, le radionucléide R considéré émet un ou plusieurs positons β+ et un ou plusieurs premiers photons γ1. Ce radionucléide est par exemple le $^{44}$Sc, mais pourrait être également $^{14}$O, $^{82}$Rb, $^{94}$Tc métastable, $^{44}$Sc métastable, $^{22}$Na, $^{48}$Va, $^{94}$Tc.

Le positon β+ interagit avec la matière environnante pour émettre deux deuxième et troisième photons γA et γB dans deux directions D2 et D3, sensiblement à 180° l'une de l'autre, les photons γA et γB ayant chacun une énergie de 511 keV. Les deux deuxième et troisième photons γA et γB sont détectés par deux premier et deuxième détecteurs 12 et 13 de la caméra 1 à tomographie par émission de positons TEP disposés de manière adéquate. Ces détecteurs font par exemple partie d'un anneau de détection des photons γA et γB, ainsi que cela est connu par l'homme du métier. La position spatiale des points 14, 15 d'impact des deuxième et troisième photons γA et γB sur les détecteurs 12 et 13 permet de calculer au cours d'une première étape S1 de calcul la ligne de réponse 16 (LOR) du radionucléide, qui est formée par les directions D2 et D3. Un scanner TEP est décrit par exemple dans le document [4].

Les interactions provoquées par le premier photon γ1 sont détectées par un télescope Compton 2. Un exemple de télescope Compton est décrit dans le document [3]. Un exemple de télescope Compton solide est décrit dans le document [5].

Le télescope Compton est utilisé afin de détecter le photon émis en coïncidence temporelle et spatiale avec le positon détecté en imagerie fonctionnelle TEP. La caméra TEP permet de localiser le lieu LOR du point d'émission sur un segment.

Suivant l'invention, un algorithme reconstitue la séquence Compton des interactions, pour pouvoir déterminer en quelles positions se trouvent les deux premières interactions provoquées par le premier photon γ1.

En effet, un télescope Compton permet de déterminer les positions des interactions Compton, mais pas leur ordre chronologique d'apparition.

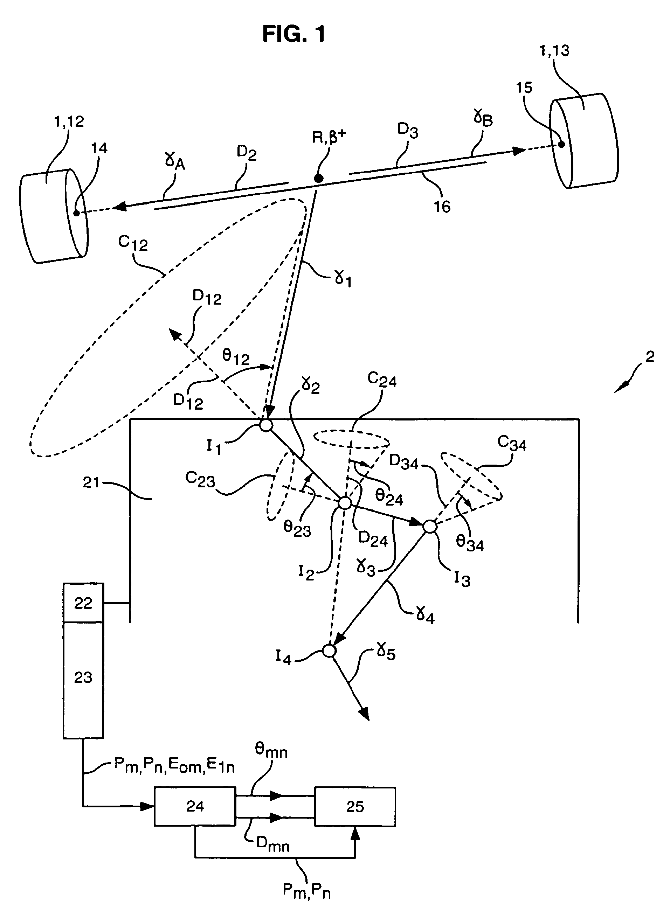

Le télescope Compton 2 comporte un milieu 21 de détection d'interactions de type Compton, formé par exemple de xénon maintenu à l'état liquide. Le premier photon incident γ1 provoque plusieurs interactions Compton en cascade dans le milieu de 21 de détection. Chaque interaction Compton provoquée par un photon incident $\gamma_n$ provoque l'émission d'un nouveau photon $\gamma_{n+1}$ et l'absorption d'énergie par un électron du milieu 21 de détection. Ce nouveau photon provoque à son tour une nouvelle interaction Compton dans le milieu 21. On a représenté à titre d'exemple illustratif à la figure 1 quatre interactions Compton successives I1, I2, I3, I4 dans le milieu 21 depuis le photon γ1, et les photons émis associés γ2, γ3, γ4, γ5. Le nombre d'interactions est par exemple d'au moins trois. Bien entendu, à la figure 1, les interactions et les lignes représentées ne sont pas forcément dans le même plan, mais sont en trois dimensions.

Le télescope Compton 2 comporte des moyens 22 de mesure de la position spatiale respective P1, P2, P3, P4 des interactions Compton I1, I2, I3, I4 dans le milieu 21 de détection, chaque position spatiale comportant les coordonnées, par exemple cartésiennes x, y, z de l'interaction.

Le télescope Compton 2 comporte des moyens 23 de mesure pour chaque interaction détectée I1, I2, I3, I4, de :

- l'énergie $E_0$ du photon incident de l'interaction,
- l'énergie $E_1$ transférée à un électron du milieu 21 de détection.

L'énergie $E_1$ transférée crée un déplacement d'électrons dans le milieu 21 de détection, ce qui permet de mesurer par les moyens 23 un courant électrique, à partir duquel on calcule la valeur de l'énergie E1 transférée. A cet effet, il est prévu des moyens de collection du courant, d'amplification et de traitement de celui-ci.

Les positions Pm des interactions Compton In et les énergies E0 et $E_1$ associées à chaque interaction In sont déterminées par le télescope Compton au cours d'une étape S2.

Pour chaque paire $I_m$, $I_n$ d'interactions parmi celles détectées, une unité 24 de calcul calcule au cours d'une étape S3 successive aux étapes S1 et S2 un angle associé mn selon la formule :

$$\cos \theta_{mn} = 1 + m_e c^2(1/E_{0m} - 1/(E_{0m} - E_{1m}))$$

où $m_e$ est la masse de l'électron, c représente la vitesse de la lumière dans le vide, $E_{0m}$ est l'énergie mesurée du photon incident de la première interaction $I_m$ de la paire, $E_{1m}$ est l'énergie transférée à un électron lors de la première interaction $I_m$ et mesurée, l'angle $\theta_{mn}$ représentant l'angle entre la direction du photon incident de la première interaction $I_m$ et l'axe $D_{mn}$ passant par la position Pm ayant été déterminée de la première interaction I et la position Pn ayant été déterminée de la deuxième interaction $I_n$ de la paire. L'axe $D_{mn}$ est orienté de la deuxième interaction $I_n$ vers la première interaction $I_m$.

Le radionucléide R se trouve sur la surface du cône géométrique C12, défini de la manière suivante en trois dimensions pour la première interaction I1 :

- sommet du cône C12 : position P1 de l'interaction I1,

- cône de révolution autour de l'axe $D_{12}$ passant par la position P1 déterminée de l'interaction I1 et la position P2 de la deuxième interaction I2,
- angle de demi-ouverture du cône C12 par rapport à son axe D12 de révolution, égal à $\theta_{12}$,
- cône C12 d'ouverture dirigée en sens inverse de I2.

Suivant l'invention, on met en oeuvre par des moyens 25 de calcul le procédé décrit ci-dessous. Les différentes étapes du procédé sont mises en oeuvre d'une manière automatique par un calculateur.

On détermine, au cours d'une étape S4 après l'étape S3, pour chaque paire ordonnée de première interaction $I_m$ et de deuxième interaction In d'interactions parmi celles détectées I1, I2, I3, I4, I5, la surface du cône géométrique Cmn, défini de la manière suivante en trois dimensions pour la première interaction Im :

- sommet du cône Cmn : position Pm de la première interaction Im de la paire ordonnée,
- cône Cmn de révolution autour de l'axe $D_{mn}$ passant par la position Pm déterminée de la première interaction Im de la paire ordonnée et la position Pn de la deuxième interaction In de la paire ordonnée,
- angle de demi-ouverture du cône Cmn par rapport à son axe $D_{mn}$ de révolution, égal à $\theta_{mn}$,
- cône Cmn d'ouverture dirigée en sens inverse de la deuxième interaction $I_n$ de la paire ordonnée.

A la figure 1, seuls les cônes C12, C23, C34 et C24, les angles de demi-ouverture associés $\theta_{12}$, $\theta_{23}$, $\theta_{34}$, $\theta_{24}$ et les axes associés D12, D23, D34, D24 ont été représentés parmi tous les arrangements possibles. Bien entendu, il y a aussi tous les autres cônes non représentés à considérer, soit pour quatre interactions, huit autres cônes.

Le nombre d'arrangements par paire d'interactions parmi les Q interactions Compton détectées dans le milieu 21 est égal à Q.(Q-1).

Au cours d'une étape S5 après l'étape S4, on examine pour chaque cône Cmn calculé si sa surface possède une intersection avec la ligne de réponse LOR 16.

On rejette les cônes Cmn, dont la surface ne s'avère pas posséder de point d'intersection avec la ligne de réponse LOR 16 à l'étape S6.

On sélectionne le cône Cmn ou les cônes Cmn, dont la surface possède un point d'intersection avec la ligne de réponse LOR 16 à l'étape S7.

Lorsqu'il n'y a qu'un seul cône Cmn dont la surface possède un point d'intersection avec la ligne de réponse LOR 16, on calcule la position de ce point d'intersection, qui représente alors la position du radionucléide R.

L'unicité de cône Cmn arrive dans plus de deux cas sur trois.

Lorsqu'il y a plusieurs cônes Cmn dont la surface possède un point d'intersection avec la ligne de réponse LOR 16, on détermine lequel de ces cônes Cmn sélectionnés est le plus probable, par exemple en utilisant l'algorithme décrit par le document [2]. On retient le cône le plus probable pour calculer la position du point d'intersection du cône Cmn retenu avec la ligne de réponse LOR 16, qui représente alors la position du radionucléide R.

Le point d'intersection détermine le lieu du radionucléide en trois dimensions. Il est solution de l'intersection du cône et du segment de réponse LOR (équation du deuxième degré ne comportant qu'une solution physique dans le champ de vue dans 90 à 95 % des cas, les 5 à 10 % restants sont rejetés à la reconstruction).

Le procédé suivant l'invention permet ainsi de multiplier la sensibilité de détection de la caméra TEP et du télescope Compton par plus de deux par rapport à l'algorithme connu par le document [2].

Une fois la position du radionucléide R déterminée, on peut exploiter celle-ci, par exemple en l'indiquant à l'utilisateur par tout moyen d'indication approprié.

Par exemple, on indique la position du radionucléide R sur une image. Cette indication de position prend par exemple la forme d'un ou de plusieurs pixels d'image ayant une apparence reconnaissable pour l'utilisateur (par exemple niveau de gris, intensité lumineuse ou couleur), différente de l'environnement où il n'a pas été détecté de radionucléide. On a ainsi un procédé d'imagerie.

Des domaines d'application de l'invention sont les suivants : pharmacologie, imagerie médicale clinique, instrumentation associée.

Une application concerne le marquage radioactif de molécules thérapeutiques, de diagnostic ou autres dans le corps humain ou animal, à des fins de suivi de celles-ci. Le radionucléide peut servir de radiotraceur. En particulier, on peut obtenir un procédé d'imagerie fonctionnelle sur le petit animal, par exemple pour l'évaluation bioclinique de biomolécules (peptides, anticorps). Par une meilleure détection des radionucléides, on réduit la dose de radioactivité à introduire, ce qui permet de minimiser les risques encourus par les manipulateurs, qui dans le cas d'utilisations extensives ne sont pas négligeables et d'éviter de biaiser le résultat de certaines évaluations précliniques (suivi de la croissance tumorale par exemple) par une irradiation des animaux dont on sait qu'elle peut avoir un impact sur le métabolisme cellulaire. La sensibilité des caméras TEP « petits animaux actuelles » est bonne mais pas excellente et les doses nécessaires pour une bonne imagerie ne sont pas totalement négligeables. Le procédé d'imagerie peut être utilisé pour des diagnostics précoces du cancer, pour assister la radio-immunothérapie béta et, plus généralement, pour envisager une réduction drastique de l'exposition à la radioactivité associée à l'imagerie fonctionnelle.

L'accès à des radioéléments émetteurs β+ comme le technétium 94, dont l'analogue 99m est largement utilisé en médecine nucléaire, doit permettre d'améliorer la résolution de l'imagerie. L'accès au rubidium 82, analogue du thallium 201, ou à des acides gras radiomarqués par l'iode 124 ou le technétium 94, devrait permettre la réalisation d'imagerie performante dans le domaine de l'ischémie cardiologique. Le développement de nouvelles approches d'imagerie telles que l'imagerie du remodelage de l'environnement tissulaire avec des inhibiteurs de métalloprotéines radioactifs, ou du ciblage des voies de l'apoptose ouvrent également dans ces domaines de nouvelles perspectives. Un autre domaine d'application pour les émetteurs de positons concerne la cancérologie et le suivi des cellules en thérapie cellulaire. Dans ce domaine, le développement de complexes lipophiles de métaux radioactifs (cuivre 64) doit permettre le suivi des cellules depuis leur injection jusqu'à leur migration sur les sites d'intérêt, soit de quelques minutes à quelques jours.

Parmi ces radioéléments, la plupart des candidats le plus souvent cités dans la littérature, iode 124, yttrium 86, rubidium 82 etc., possèdent des émissions photoniques de haute énergie en plus de l'émission β+. Ces émissions sont la source d'une dégradation de l'image TEP, imposent des contraintes supplémentaires en termes de radioprotection et augmentent le risque potentiel à long terme d'un usage répété de la TEP.

La conception d'une nouvelle caméra, basée sur la détection de 3 photons, permet une imagerie sans reconstruction avec des quantités de radioactivité bien plus faibles et des temps d'acquisition plus courts que ceux utilisés habituellement. Dans toutes ces applications l'imagerie 3 photons pourrait s'appliquer avec l'avantage des faibles doses pour des examens appelés à être réalisés beaucoup plus fréquemment que par le passé et d'une plus grande rapidité d'acquisition. Tous les émetteurs β+ n'émettent pas de façon simultanée un rayonnement gamma d'énergie utilisable, mais c'est le cas de certains d'entre eux (technétium 94, scandium 44, oxygène 14, ...) qui pourront être produits et substitués aux précédents pour les applications d'imagerie à 3 photons.

Le xénon liquide est reconnu depuis longtemps comme étant un milieu particulièrement approprié pour la détection de particules corpusculaires (leptons, γ, hadrons, particules super-symétriques).

Liste des références citées

[1] Nuclear medical imaging using β+γ coincidences from 44Sc radionuclide with liquid xénon as detection medium, de C. Grignon, J. Barbet, M. Bardiès, T. Carlier, J.F. Chatal, O. Couturier, J.P. Cussonneau, A. Faivre, L. Ferrer, S. Girault, T.Haruyama, P. Le Ray, L. Luquin, S. Lupone, V. Métivier, E. Morteau, N. Servagent, D. Thers, Nuclear Instruments and Methods in Physics Research A 571 (2007), pages 142-145.

[2] Compton scattering sequence reconstruction algorithm for the liquid xénon gamma-ray imaging telescope (LXeGRIT), de U.G. Oberlack, E. Aprile, A. Curioni, V. Egorov, K.L. Giboni, Columbia Astrophysics Laboratory, Columbia University, New York, USA, arXiv:astroph/0012296v1, 13 Dec 2000.

[3] A study of the LXeGRIT detection efficiency for MeV gamma-rays during the 2000 balloon flight campaign, de A. Curioni, E0. Aprile, T. Doke, K.L. Giboni, M. Kobayashi, U.G. Oberlack, Nuclear Instruments and Methods in Physics Research A 576 (2007), pages 350-361.

[4] Time of Flight in PET Revisited, de W. W. Moses, IEEE Transactions on Nuclear Science, Vol. 50, N° 5, octobre 2003, pages 1325-1330.

[5] The Nuclear Compton Telescope (NCT) : Scientific goals and expected sensivity, de Hsiang-Kuang Chang, Steven Boggs, Yuan-Hann Chang, for the NCT collaboration, Advances in Space Research 40 (2007), pages 1281-1287.

**Revendications**

1. Procédé de localisation de la position d'un radionucléide émetteur de positons et dont le noyau fils émet au moins un photon par désexcitation, dans lequel

   on détecte par une caméra (1, 12, 13) à tomographie par émission de positons une ligne de réponse du radionucléide, la position du radionucléide étant située à l'intersection entre la ligne (16) de réponse et un cône géométrique (C12) déterminé, le cône (C12) ayant pour sommet la position d'une interaction (I1) de type Compton à partir du photon (y1) émis par désexcitation du noyau fils du radionucléide, et pour demi-ouverture l'angle (θ) entre la direction du photon incident de l'interaction (I1) et l'axe de révolution du cône formé par la direction (D12) joignant cette interaction (I1) et la position d'une autre interaction (I2),

un télescope Compton (2) est utilisé pour détecter une pluralité, supérieure ou égale à deux, d'interactions (I1, I2, I3, I4) de type Compton provoquées en cascade dans un milieu (21) de détection du télescope Compton à partir du photon émis par désexcitation du noyau fils du radionucléide,

on mesure par le télescope Compton (2) la position de chaque

interaction (Pm, Pn) de ladite pluralité,

**caractérisé en ce que**

pour chacun de la multiplicité des arrangements ordonnés par paire de première et deuxième interactions (Im, In) de type Compton parmi la pluralité d'interactions détectées, on détermine par le télescope Compton (2) l'angle ($\theta_{mn}$) entre la direction du photon incident de la première interaction (Im) et l'axe géométrique joignant la position (Pm) de la première interaction (Im) et la position (Pn) de la deuxième interaction (In) et on reconstruit la surface géométrique du cône (Cmn) ayant pour sommet la position (Pm) de la première interaction (Im) et pour demi-ouverture ledit angle ($\theta_{mn}$) déterminé autour dudit axe géométrique, ledit axe géométrique formant l'axe de révolution du cône (Cmn) et étant orienté dans le sens allant de la deuxième interaction (In) vers la première interaction (Im), on élimine les cônes (Cmn) dont la surface géométrique ne possède pas d'intersection avec la ligne de réponse (16), on sélectionne au moins un cône (Cmn) dont la surface géométrique possède une intersection avec la ligne (16) de réponse,

on sélectionne la position du radionucléide à partir de ladite intersection reconstruite de la ligne de réponse (16) avec la surface géométrique du cône sélectionné (Cmn).

2. Procédé suivant la revendication 1, **caractérisé en ce que**
   on détermine par le télescope Compton ledit angle $\theta_{mn}$ pour chaque arrangement par paire suivant la formule :

$$\cos \theta_{mn} = 1 + m_e c^2 (1/E_{0m} - 1/(E_{0m} - E_{1m}))$$

où $m_e$ est la masse de l'électron, c représente la vitesse de la lumière dans le vide, $E_{0m}$ est l'énergie mesurée du photon incident de la première interaction ($I_m$) de la paire, $E_{1m}$ est l'énergie transférée à un électron lors de la première interaction ($I_m$) et mesurée.

3. Procédé suivant l'une quelconque des revendications précédentes, **caractérisé en ce que** on examine si il n'y a qu'un seul cône (Cmn) sélectionné dont la surface possède un point d'intersection avec la ligne de réponse (16), et, dans l'affirmative, on calcule la position du radionucléide (R) comme étant la position de ce point d'intersection de ce seul cône sélectionné dont la surface possède un point d'intersection avec la ligne de réponse (16).

4. Procédé suivant l'une quelconque des revendications précédentes, **caractérisé en ce que** on examine si il y a plusieurs cônes (Cmn) sélectionnés dont la surface possède un point d'intersection avec la ligne de réponse (16), et, dans l'affirmative, on détermine lequel de ces cônes (Cmn) sélectionnés dont la surface possède un point d'intersection avec la ligne de réponse (16) est le plus probable, on calcule la position du radionucléide (R) comme étant la position du point d'intersection du cône sélectionné, dont la surface possède un point d'intersection avec la ligne de réponse (16) et qui est le plus probable.

5. Application du procédé suivant l'une quelconque des revendications 1 à 4 à l'imagerie de localisation du radionucléide.

6. Application du procédé suivant l'une quelconque des revendications 1 à 4 à la localisation d'au moins un marqueur radioactif comprenant ledit radionucléide.

7. Application suivant la revendication 6 à la localisation d'au moins un marqueur radioactif, comprenant le radionucléide, pour localiser dans un milieu géologique une substance radiomarquée par le marqueur.

8. Application suivant la revendication 7, dans laquelle la substance radiomarquée est de l'eau.

9. Application du procédé suivant l'une quelconque des revendications 1 à 4 à la détection de fuites d'un container contenant au moins le radionucléide.

**10.** Application du procédé suivant l'une quelconque des revendications 1 à 4 à la détection d'un objet contenant au moins le radionucléide.

**11.** Dispositif pour la mise en oeuvre du procédé suivant l'une quelconque des revendications 1 à 4, comprenant une caméra (1, 12, 13) à tomographie par émission de positons pour détecter une ligne de réponse du radionucléide, un télescope Compton (2) comportant :

- des moyens pour détecter une pluralité, supérieure ou égale à deux, d'interactions (I1, I2, I3, I4) de type Compton provoquées en cascade dans un milieu (21) de détection du télescope Compton à partir du photon émis par desexcitation du noyau fils du radionucléide,
- des moyens (22) de mesure de la position de chaque interaction (Pm, Pn) de ladite pluralité dans le milieu (21) de détection,

le dispositif étant **caractérisé en ce qu'**il comporte, en plus de la caméra et du télescope :

- des moyens (23, 24) agencés pour déterminer, pour chacun de la multiplicité des arrangements ordonnés par paire de première et deuxième interactions (Im, In) de type Compton parmi la pluralité d'interactions détectées, l'angle (θmn) entre la direction du photon incident de la première interaction (Im) et l'axe géométrique joignant la position (Pm) de la première interaction (Im) et la position (Pn) de la deuxième interaction (In), pour reconstruire la surface géométrique du cône (Cmn) ayant pour sommet la position (Pm) de la première interaction (Im) et pour demi-ouverture ledit angle ($\theta_{mn}$) déterminé autour dudit axe géométrique, ledit axe géométrique formant l'axe de révolution du cône (Cmn) et étant orienté dans le sens allant de la deuxième interaction (In) vers la première interaction (Im),
- des moyens (25) agencés pour éliminer les cônes (Cmn) dont la surface géométrique ne possède pas d'intersection avec la ligne de réponse (16),
- des moyens (25) agencés pour sélectionner au moins un cône (Cmn) dont la surface géométrique possède une intersection avec la ligne (16) de réponse,
- des moyens (25) agencés pour sélectionner la position du radionucléide à partir de ladite intersection reconstruite de la ligne de réponse (16) avec la surface géométrique du cône sélectionné (Cmn).

**Claims**

**1.** A process for locating the position of a radionuclide emitting positions and whereof the child core emits at least one photon by de-excitation, in which
a response line of the radionuclide is detected by a camera (1, 12, 13) with tomography by positon emission,
the position of the radionuclide being situated at the intersection between the response line (16) and a determined geometric cone (C12), the cone (C12) having as apex the interaction position (I1) of Compton type from the photon (γ1) emitted by de-excitation of the child core of the radionuclide, and as semi-opening the angle (θ) between the direction of the incident photon of the interaction (I1) and the axis of revolution of the cone formed by the direction (D12) joining this interaction (I1) and the position of another interaction (I2),
a Compton telescope (2) is used for detecting a plurality, greater than or equal to two, of interactions (I1, I2, I3, I4) of Compton type caused in cascade in a detection medium (21) of the Compton telescope from the photon emitted by de-excitation of the child core of the radionuclide,
the position of each interaction (Pm, Pn) of said plurality is measured by the Compton telescope (2),
**characterised in that**
for each of the multiplicity of the arrangements ordered per pair of first and second interactions (Im, In) of Compton type among the plurality of detected interactions, the angle ($\theta_{mn}$) between the direction of the incident photon of the first interaction (Im) and the geometric axis joining the position (Pm) of the first interaction (Im) and the position (Pn) of the second interaction (In) is determined by the Compton telescope (2) and the geometric surface of the cone (Cmn) having for apex the position (Pm) of the first interaction (Im) and for semi-opening said angle ($\theta_{mn}$) determined around said geometric axis is reconstructed, said geometric axis forming the axis of revolution of the cone (Cmn) and being oriented in the direction going from the second interaction (In) to the first interaction (Im),
those cones (Cmn) are eliminated whereof the geometric surface has no intersection with the response line (16),
at least one cone (Cmn) is selected whereof the geometric surface has an intersection with the response line (16),
the position of the radionuclide is selected from said reconstructed intersection of the response line (16) with the geometric surface of the selected cone (Cmn).

2. The process as claimed in Claim 1, **characterised in that** said angle $\theta_{mn}$ is determined by Compton telescope for each arrangement per pair as per the formula:

$$\cos\ \theta_{mn}\ =\ 1\ +\ m_e c^2 (1/E_{0m} - 1/(E_{0m}\ -\ E_{1m}))$$

where $m_e$ is the mass of the electron, c represents the speed of light in vacuum, $E_{0m}$ is the measured energy of the incident photon of the first interaction (Im) of the pair, $E_{1m}$ is the energy transferred to an electron during the first interaction (Im) and measured.

3. The process as claimed in any one of the preceding claims, **characterised in that** an examination is made as to whether there is only a single cone (Cmn) selected whereof the surface has a point of intersection with the response line (16), and, in the affirmative, the position of the radionuclide (R) is calculated as being the position of this point of intersection of this single selected cone whereof the surface has a point of intersection with the response line (16).

4. The process as claimed in any one of the preceding claims, **characterised in that** an examination is made as to whether there are several selected cones (Cmn) whereof the surface has a point of intersection with the response line (16), and, in the affirmative, it is determined which of these selected cones (Cmn) whereof the surface has a point of intersection with the response line (16) is the most probable, the position of the radionuclide, (R) is calculated as being the position of the point of intersection of the selected cone, whereof the surface has a point of intersection with the response line (16) and which is the most probable.

5. Application of the process as claimed in any one of Claims 1 to 4 to location imagery of the radionuclide.

6. Application of the process as claimed in any one of Claims 1 to 4 to locating at least one radioactive marker comprising said radionuclide.

7. Application as claimed in Claim 6 to locating at least one radioactive marker, comprising the radionuclide, to locate a radiomarked substance by the marker in a geological medium.

8. Application as claimed in Claim 7, in which the radicmarked substance is water.

9. Application of the process as claimed in any one of Claims 1 to 4 to detection of leaks from a container containing at least the radionuclide.

10. Application of the process as claimed in any one of Claims 1 to 4 to detection of an object containing at least the radionuclide,

11. A device for executing the process as claimed in any one of Claims 1 to 4, comprising a positon emission tomography camera (1, 12, 13) for detecting a response line of the radionuclide, a Compton telescope (2) comprising;

- means for detecting a plurality, greater than or equal to two, of interactions (I1, I2, I3, I4) of Compton type caused in cascade in a detection medium (21) of the Compton telescope from the photon emitted by de-excitation of the child core of the radionuolide,
- means (22) for measuring the position of each interaction (Pm, Pn) of said plurality in the detection medium (21), the device being **characterised in that** it comprises, in addition to the camera and the telescope:
- means (23, 24) for determining, for each of the multiplicity of arrangements ordered per pair of first and second interactions (Im, In) of Compton type among the plurality of interactions detected, the angle ($\theta_{mn}$) between the direction of the incident photon of the first interaction (Im) and the geometric axis joining the position (Pm) of the first interaction (Im) and the position (Pn) of the second interaction (In), for reconstructing the geometric surface of the cone (Cmn) having for apex the position (Pm) of the first interaction (Im) and for semi-opening said angle ($\theta_{mn}$) determined around said geometric axis, said geometric axis forming the axis of revolution of the cone (Cmn) and being oriented in the direction going from the second interaction (In) to the first interaction (Im),
- means (25) arranged for eliminating the cones (Cmn) whereof the geometric surface has no intersection with the response line (16),
- means (25) arranged for selecting at least one cone (Cmn) whereof the geometric surface has an interaction

9

with the response line (16),
- means (25) arranged for selecting the position of the radionuclide from said reconstructed intersection of the response line (16) with the geometric surface of the selected cone (Cmn).

**Patentansprüche**

1. Verfahren zum Lokalisieren der Position eines Positronen emittierenden Radionuklids, dessen Tochterkern zumindest ein Photon durch Entregung emittiert, wobei
   mit einer Kamera (1, 12, 13) für Positronen-Emissions-Tomographie eine Antwortline des Radionuklids erfasst wird, die Position des Radionuklids am Schnittpunkt zwischen der Antwortlinie (16) und einem bestimmten geometrischen Kegel (C12) liegt, wobei der Kegel (C12) mit seiner Spitze die Position einer Interaktion (I1) vom Typ Compton ausgehend vom Photon (γ1), das durch Entregung des Tochterkerns des Radionuklids emittiert wurde, einnimmt, und bei halber Strahlenbreite den Winkel (θ) zwischen der Richtung des einfallenden Photons der Interaktion (I1) und der Drehachse des Kegels einnimmt, die durch die Richtung (D12) gebildet wird, die auf diese Interaktion (I1) und auf die Position einer weiteren Interaktion (I2) trifft,
   wobei ein Compton-Teleskop (2) verwendet wird, um eine Mehrzahl größer oder gleich zwei von Interaktionen (I1, I2, I3, I4) vom Typ Compton zu erfassen, die als Kaskade in einem Erfassungsmilieu (21) des Compton-Teleskops ausgehend vom Photon hervorgerufen wurden, das durch Entregung des Tochterkerns des Radionuklids emittiert wurde,
   mit dem Compton-Teleskop (2) die Position einer jeden Interaktion (Pm, Pn) der Mehrzahl gemessen wird,
   **dadurch gekennzeichnet, dass**
   für jede aus der Vielfalt von paarweise geordneten Anordnungen von erster und zweiter Interaktion (Im, In) vom Typ Compton unter der Mehrzahl von erfassten Interaktionen durch das Compton-Teleskop (2) der Winkel (θ$_{mn}$) zwischen der Richtung des einfallenden Photons der ersten Interaktion (Im) und der geometrischen Achse, die auf die Position (Pm) der ersten Interaktion (Im) und auf die Position (Pn) der zweiten Interaktion (In) trifft, bestimmt wird und die geometrische Fläche des Kegels (Cmn) rekonstruiert wird, der mit seiner Spitze die Position (P$_m$) der ersten Interaktion (im) einnimmt und bei halber Strahlenbreite den um die geometrische Achse bestimmten Winkel (θ$_{mn}$) einnimmt, wobei die geometrische Achse die Drehachse des Kegels (Cmn) bildet und in Richtung von der zweiten Interaktion (In) zur ersten Interaktion (Im) hin ausgerichtet ist,
   die Kegel (Cmn) eliminiert werden, deren geometrische Fläche keinen Schnittpunkt mit der Antwortlinie (16) besitzt, und dass zumindest ein Kegel (Cmn) ausgewählt wird, dessen geometrische Fläche einen Schnittpunkt mit der Antwortlinie (16) besitzt,
   die Position des Radionuklids ausgehend von dem rekonstruierten Schnittpunkt der Antwortlinie (16) mit der geometrischen Fläche des ausgewählten Kegels (Cmn) ausgewählt wird.

2. Verfahren nach Anspruch 1, **dadurch gekennzeichnet, dass** mit dem Compton-Teleskop der Winkel θ$_{mn}$ für jede paarweise Anordnung bestimmt wird nach der Formel:

$$\cos \theta_{mn} = 1 + m_e c^2 \, (1/E_{0m} - 1/(E_{0m} - E_{1m}))$$

worin m$_e$ die Masse des Elektrons ist, c die Lichtgeschwindigkeit im Vakuum darstellt, E$_{0m}$ die gemessene Energie des einfallenden Photons der ersten Interaktion (Im) des Paares ist, E$_{1m}$ die bei der ersten Interaktion (Im) auf ein Elektron übertragene und gemessene Energie ist.

3. Verfahren nach einem der vorangehenden Ansprüche, **dadurch gekennzeichnet, dass** untersucht wird, ob es nur einen ausgewählten Kegel (Cmn) gibt, dessen Fläche einen Schnittpunkt mit der Antwortlinie (16) besitzt und, falls ja, die Position des Radionuklids (R) als Position dieses Schnittpunkts dieses einzigen ausgewählten Kegels berechnet wird, dessen Fläche einen Schnittpunkt mit der Antwortlinie (16) besitzt.

4. Verfahren nach einem der vorangehenden Ansprüche, **dadurch gekennzeichnet, dass** untersucht wird, ob es mehrere ausgewählte Kegel (Cmn) gibt, deren Fläche einen Schnittpunkt mit der Antwortlinie (16) besitzt, und, falls ja, bestimmt wird, welcher dieser ausgewählten Kegel (Cmn), deren Fläche einen Schnittpunkt mit der Antwortlinie (16) besitzt, der wahrscheinlichste ist, und die Position des Radionuklids (R) als Position dieses Schnittpunkts des ausgewählten Kegels berechnet wird, dessen Fläche einen Schnittpunkt mit der Antwortlinie (16) besitzt und der am wahrscheinlichsten ist.

**5.** Anwendung des Verfahrens nach einem der Ansprüche 1 bis 4 bei der Abbildung zur Lokalisierung des Radionuklids.

**6.** Anwendung des Verfahrens nach einem der Ansprüche 1 bis 4 zur Lokalisierung von zumindest einem radioaktiven Marker, der das Radionuklid enthält.

**7.** Anwendung nach Anspruch 6 zur Lokalisierung zumindest eines radioaktiven Markers, der das Radionuklid enthält, um in einem geologischen Milieu eine mit dem Marker radiomarkierte Substanz zu lokalisieren.

**8.** Anwendung nach Anspruch 7, wobei die radiomarkierte Substanz Wasser ist.

**9.** Anwendung des Verfahrens nach einem der Ansprüche 1 bis 4 bei der Erfassung von Lecks bei einem Behälter, der zumindest das Radionuklid enthalt.

**10.** Anwendung des Verfahrens nach einem der Ansprüche 1 bis 4 bei der Erfassung eines Objekts, das zumindest das Radionuklid enthält.

**11.** Vorrichtung zum Durchführen des Verfahrens nach einem der Ansprüche 1 bis 4, enthaltend
eine Kamera (1, 12, 13) für Positronen-Emissions-Tomographie zum Erfassen einer Antwortlinie des Radionuklids,
ein Compton-Teleskop (2) mit:

- Mitteln zum Erfassen einer Mehrzahl größer oder gleich zwei von Interaktionen (I1, I2, I3, I4) vom Typ Compton, die als Kaskade in einem Erfassungsmilieu (21) des Compton-Teleskops ausgehend vom Photon hervorgerufen wurden, das durch Entregung des Tochterkerns des Radionukiids emittiert wurde,
- Mitteln (22) zum Messen der Position einer jeden Interaktion (Pm, Pn) der genannten Mehrzahl in dem Erfassungsmilieu (21),

wobei die Vorrichtung **dadurch gekennzeichnet ist, dass** sie neben der Kamera und dem Teleskop aufweist:

- Mittel (23, 24), die geeignet sind, für jede aus der Vielfalt von paarweise geordneten Anordnungen von erster und zweiter Interaktion (Im, In) vom Typ Compton unter der Mehrzahl von erfassten Interaktionen den Winkel ($\theta_{mn}$) zwischen der Richtung des einfallenden Photons der ersten Interaktion (Im) und der geometrischen Achse zu bestimmen, die auf die Position (Pm) der ersten Interaktion (Im) und auf die Position (Pn) der zweiten Interaktion (In) trifft, um die geometrische Fläche des Kegels (Cmn) zu rekonstruieren, der mit seiner Spitze die Position (Pm) der ersten Interaktion (Im) einnimmt und bei halber Strahlenbreite den Winkel ($\theta_{mn}$) einnimmt, der um die geometrische Achse bestimmt wurde, wobei die geometrische Achse die Drehachse des Kegels (Cmn) bildet und in Richtung von der zweiten Interaktion (In) zur ersten Interaktion (Im) hin ausgerichtet ist,
- Mittel (25), die geeignet sind, die Kegel (Cmn) zu eliminieren, deren geometrische Fläche keinen Schnittpunkt mit der Antwortlinie (16) besitzt,
- Mittel (25), die geeignet sind, zumindest einen Kegel (Cmn) auszuwählen, dessen geometrische Fläche einen Schnittpunkt mit der Antwortlinie (16) besitzt,
- Mittel (25), die geeignet sind, die Position des Radionuklids ausgehend von dem rekonstruierten Schnittpunkt der Antwortlinie (16) mit der geometrischen Fläche des ausgewählten Kegels (Cmn) auszuwählen.

# FIG. 1

# FIG. 2

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6484051 B1

**Littérature non-brevet citée dans la description**

- **C. GRIGNON ; J. BARBET ; M. BARDIÈS ; T. CARLIER ; J.F. CHATAL ; O. COUTURIER ; J.P. CUSSONNEAU ; A. FAIVRE ; L. FERRER ; S. GI-RAULT.** Nuclear medical imaging using β+γ coincidences from Sc radionuclide with liquid xénon as detection medium. *Nuclear Instruments and Methods in Physics Research A,* 2007, vol. 571, 142-145
- **U.G. OBERLACK ; E. APRILE ; A. CURIONI ; V. EGOROV ; K.L. GIBONI.** Compton scattering sequence reconstruction algorithm for the liquid xénon gamma-ray imaging telescope (LXeGRIT. *Columbia Astrophysics Laboratory,* 13 Décembre 2000
- **A. CURIONI ; E0. APRILE ; T. DOKE ; K.L. GIBONI ; M. KOBAYASHI ; U.G. OBERLACK.** A study of the LXeGRIT detection efficiency for MeV gamma-rays during the 2000 balloon flight campaign. *Nuclear Instruments and Methods in Physics Research A,* 2007, vol. 576, 350-361
- **W. W. MOSES.** Time of Flight in PET Revisited. *IEEE Transactions on Nuclear Science,* Octobre 2003, vol. 50 (5), 1325-1330
- **HSIANG-KUANG CHANG ; STEVEN BOGGS ; YUAN-HANN CHANG.** The Nuclear Compton Telescope (NCT) : Scientific goals and expected sensivity. *for the NCT collaboration, Advances in Space Research,* 2007, vol. 40, 1281-1287